(19) Europäisches Patentamt
European Patent Office
Office européen des brevets

(11) Numéro de publication: **0 037 318**
**B1**

(12) # FASCICULE DE BREVET EUROPEEN

(45) Date de publication du fascicule du brevet: **06.04.83**

(51) Int. Cl.³: **A 61 K 7/06**, A 61 K 7/48

(21) Numéro de dépôt: **81400445.3**

(22) Date de dépôt: **20.03.81**

(54) **Nouvelles compositions utiles notamment dans le traitement et la prévention des pellicules et contenant des dérivés métalliques de l'acide thiobenzoique.**

(30) Priorité: **21.03.80 FR 8006369**

(43) Date de publication de la demande:
**07.10.81 Bulletin 81/40**

(45) Mention de la délivrance du brevet:
**06.04.83 Bulletin 83/14**

(84) Etats contractants désignés:
**AT BE CH DE FR GB IT LI LU NL SE**

(56) Documents cités:
**FR-A-1 594 624**
**EXPERIENTIA, vol. 22, no. 5, 15 mai 1966 Basel, CH A.D. INGLOT et al.: "Effect of choline salicylates and some other analogues of salicylic acid on the replication of EMC virus in vitro" pages 322-324**

(73) Titulaire: **PIERRE FABRE S.A., 125, rue de la Faisanderie, F-75116 Paris (FR)**

(72) Inventeur: **Cousse, Henri, Dr. Chem., La Foun de los Nobios Chemin de Lastinos, F-81100 Castres (FR)**
Inventeur: **Mouzin, Gilbert, Dr. Chem., 21, rue Sainte-Foy, F-81100 Castres (FR)**
Inventeur: **Cassan André, 108, rue des Fontaines, F-31300 Toulouse (FR)**
Inventeur: **Ribet, Jean-Paul, 147, rue Goya, F-81100 Castres (FR)**

(74) Mandataire: **Corre, Jacques Denis Paul et al, Cabinet Regimbeau 26, Avenue Kléber, F-75116 Paris (FR)**

EP 0 037 318 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

# Nouvelles compositions utiles notamment dans le traitement et la prévention des pellicules et contenant des dérivés métalliques de l'acide thiobenzoïque

La présente invention, réalisée au Centre de Recherches Pierre Fabre, concerne de nouvelles compositions destinées au traitement et à la prévention des désordres cutanés et notamment au traitement et à la prévention des pellicules.

La présente invention concerne, en particulier, une composition à usage externe pour le traitement et la prévention des désordres cutanés, notamment le traitement et la prévention des pellicules du cuir chevelu, caractérisée en ce qu'elle comporte à titre de principe actif au moins un dérivé métallique de formule:

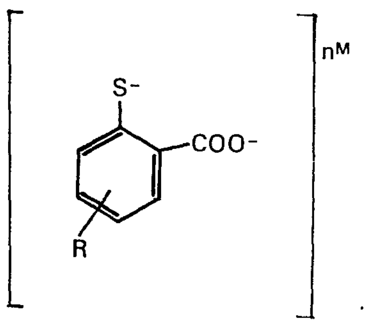

dans laquelle,
R est l'hydrogène, un halogène, un radical alcoyle ou alcoxy;
M est un cation métallique;
n est un nombre entier ou fractionnaire dont la valeur est telle que le dérivé de formule I soit neutre électriquement.

Parmi les halogènes qui peuvent être présents à titre de substituant, il faut citer le fluor, le chlore, le brome et l'iode mais l'on préfère le chlore.

Par «radical alcoyle» il faut entendre les radicaux alcoyles inférieurs en $C_1$ à $C_3$ et surtout le radical méthyle.

Par «radical alcoxy» il faut entendre les radicaux alcoxy inférieurs en $C_1$ à $C_3$ et surtout le radical méthoxy.

M est un cation métallique d'un métal compatible, c'est-à-dire non toxique et ayant une bonne tolérance cutanée choisi parmi: $Ag^+$, dans ce cas $n = 2$ et $Zn^{++}$, $Cu^{++}$, $Mn^{++}$, $Fe^{++}$, $Ni^{++}$, $Mg^{++}$, dans ce cas $n = 1$.

Ainsi, parmi les dérivés métalliques de formule I, il faut citer plus particulièrement les dérivés suivants:
- le thiosalicylate de zinc,
- le thiosalicylate de manganèse,
- le thiosalicylate de nickel,
- le thiosalicylate de fer,
- le thiosalicylate d'argent,
- le thiosalicylate de cuivre,
- l'ortho thiocrésotinate de zinc,
- le méthoxy-4 thiosalicylate de zinc,
- le chloro-5 thiosalicylate de zinc.

Les dérivés de formule I peuvent être préparés par des procédés connus, en particulier par action d'un sel du métal correspondant à M sur l'acide thiosalicylique ou le dérivé de cet acide correspondant, sous forme de sel soluble de sodium par exemple, le dérivé de formule I cristallise à partir de la solution.

Les compositions pour usage externe selon la présente invention sont plus particulièrement des compositions cosmétologiques, notamment des compositions antipelliculaires, antiséborrhéiques et pour la prévention du vieillissement cutané. Mais, les compositions selon la présente invention sont également utilisables pour le traitement et la prévention d'autres troubles cutanés tels que l'acné par exemple.

Ces compositions peuvent se présenter sous n'importe quelle forme utilisable par voie externe, par exemple: crème, pommade, gel, lotion, spray, suivant l'application envisagée. Lorsque le but recherché est une action antipelliculaire, la composition sera de préférence un shampooing ou une lotion capillaire.

Les différents excipients pouvant entrer dans les compositions selon la présente invention sont choisis parmi les excipients connus, notamment dans le domaine cosmétologique, et l'on donnera ci-après des exemples de telles compositions.

La présente invention concerne également l'application des composés de formule I pour le traitement et la prévention des désordres cutanés et en particulier à titre d'agent antipelliculaire.

Les exemples suivants sont destinés à illustrer la préparation de certains des composés selon la présente invention.

Exemple 1:

Préparation du thiosalicylate de zinc

A une solution de 38,54 g (0,25 mole) d'acide thiosalicylique dans 500 $cm^3$ de soude N (0,5 mole) on ajoute sous bonne agitation une solution de 71,88 g (0,25 mole) de sulfate de zinc heptahydrate dans 250 $cm^3$ d'eau.

Le mélange réactionnel cristallise, on agite 15 minutes, on filtre et on lave à l'eau jusqu'à élimination des ions sulfates. On sèche à l'étuve à 100°C, on récupère 48 g de cristaux beiges (rendement 90%) de produit de formule

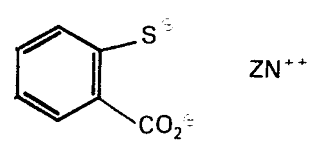

Formule brut: $C_7 H_4 O_2 S Zn$
Masse moléculaire: 217,54
Cristaux: beiges
Point de fusion: > à 300°C.

Exemple 2

Préparation du thiosalicylate de manganèse

D'une façon similaire à celle décrite dans l'exemple 1, mais en utilisant le chlorure de manganèse tétrahydrate, on obtient le produit de formule:

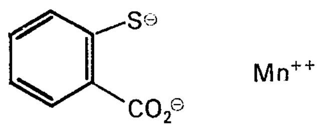

Exemple 3

Préparation du thiosalicylate de nickel

D'une façon similaire à celle décrite dans l'exemple 1, mais en utilisant le chlorure de nickel, on obtient le produit de formule:

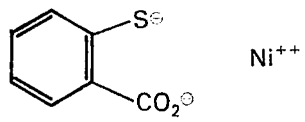

Exemple 4

Préparation du thiosalicylate de fer

D'une façon similaire à celle décrite dans l'exemple 1, mais en utilisant le sulfate de fer, on obtient le produit de formule:

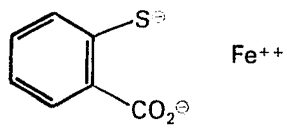

Exemple 5

Préparation du thiosalicylate d'argent

D'une façon similaire à celle décrite dans l'exemple 1, mais en utilisant le nitrate d'argent, on obtient le produit de formule:

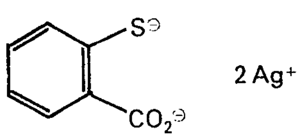

Exemple 6

Préparation du thiosalicylate de cuivre

D'une façon similaire à celle décrite dans l'exemple 1, mais en utilisant le sulfate de cuivre pentahydrate, on obtient le produit de formule:

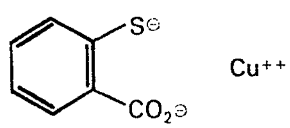

Exemple 7

Préparation de l'ortho thiocrésotinate de zinc

D'une façon similaire à celle décrite dans l'exemple 1, mais en utilisant l'acide ortho thiocrésotinique et le sulfate de zinc, on obtient le produit de formule:

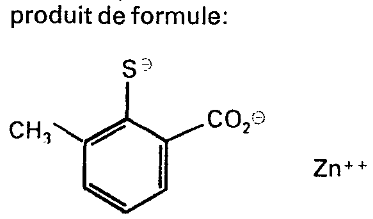

Exemple 8

Préparation du méthoxy-4-thiosalicylate de zinc

D'une façon similaire à celle décrite dans l'exemple 1, mais en utilisant l'acide méthoxy-4-thiosalicylique et le sulfate de zinc, on obtient le produit de formule:

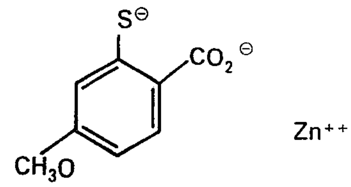

Exemple 9

Préparation du chloro-5-thiosalicylate de zinc

D'une façon similaire à celle décrite dans l'exemple 1, mais en utilisant l'acide chloro-5-thiosalicylique et le sulfate de zinc, on obtient le produit de formule:

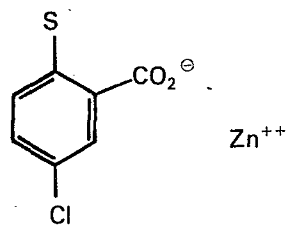

Afin de mettre en évidence les propriétés antipelliculaires avantageuses des composés selon la présente invention, on utilise à titre de composé de référence le produit utilisé couramment dans les préparations cosmétiques antipelliculaires: la pyrithione zinc ou omadine zinc de formule:

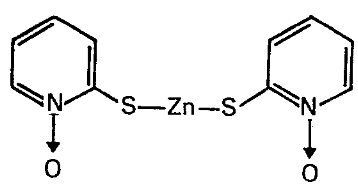

$C_{10}H_8N_2O_2S_2Z_n$
masse: 317

1. Pouvoir anti-oxydant

Cette propriété est importante dans l'étude d'un agent antipelliculaire car il a été démontré que l'une des causes d'apparition de pellicules provient de phénomènes d'oxydation qui peuvent être diminués en présence d'agents anti-oxydants.

La mesure du pouvoir anti-oxydant est effectuée selon la méthode décrite par Carnat et Pourrat (Ann. Pharm. Fr. 1979, 37, no 3–4, p. 119–124) en remplaçant l'huile de lin par de l'acide linoléique.

L'oxydation de l'acide linoléique est suivie chaque jour par une mesure de l'indice de peroxydes Ip.

a) Indice de peroxydes

Dans un flacon contenant l'acide linoléique on ajoute 15 ml de $CHCl_3$, 20 ml d'acide acétique RP puis 1 ml de solution aqueuse saturée de KI. On agite 1 mn et on laisse reposer le flacon 5 mn à l'obscurité. Après avoir ajouté 100 ml d'eau on dose l'iode libéré par du thiosulfate de sodium 0,01 N.

$Ip = (v-v_o) \times 5$

v = volume de $Na_2S_2O_3$ versé dans le dosage
$v_o$ = volume de $Na_2S_2O_3$ versé dans l'essai à blanc
Ip est exprimé en mmoles d'oxygène/kg d'acide gras.

Remarque: $v_o$ doit être voisin de O car un Ip élevé révèle une oxydation du KI par l'oxygène de l'air ou dissous dans les réactifs. Pour éviter cela, les réactifs et le solvant devront être débarrassés de l'oxygène dissous par barbotage de gaz inerte, ceci afin d'obtenir des résultats comparables.

Les différents agents anti-oxydants étudiés sont mis en suspension dans l'acide linoléique et les mélanges ainsi obtenus sont stockés à l'étuve (60°C).

Tous les jours un Ip est effectué sur les mélanges suivants:

1 - acide thiosalicylique à 1%
2 - pyridine thione Zn à 1%
3 - thiosalicylate de Zn à 1%.

Les résultats observés sont rassemblés dans le tableau I ci-après:

Tableau I
Indices de peroxydes (en millimoles $D_2$ pour 1000 g d'acide linoléique)

| | 0 | 1 jour | 2 jours | 3 jours | 4 jours |
|---|---|---|---|---|---|
| Acide linoléique (essai témoin) | 3,2 | 144 | 203,7 | 210,2 | 209 |
| Acide linoléique + acide thiosalicylique | 0,8 | 117,2 | 191,9 | 202,7 | 204,2 |
| Acide linoléique + pyrithione zinc | 0,7 | 98 | 174,1 | 177,6 | 183,7 |
| Acide linoléique + thiosalicylate de zinc | 0,6 | 45,5 | 84,4 | 99,5 | 103,9 |

Les essais précédents démontrent l'excellente activité anti-oxydante du thiosalicylate de zinc selon la présente invention par rapport à la pyrithione zinc.

b) Mesure du retard d'auto-oxydation

Selon L. Waginaire (Bulletin technique SFPA no 60) l'évolution des peroxydes se fait en trois phases:
1. évolution lente de l'Ip (non observée avec l'acide linoléique)
2. évolution rapide de l'Ip (deux premiers jours)
3. stabilisation et décroissance de l'Ip.

Cette dernière période apparaît après plusieurs semaines. L'auteur attribue ce phénomène à la formation de groupements carbonyles (aldéhydes et cétones).

L'étude précédente a été remaniée en utilisant les conditions opératoires fixées par Waginaire, c'est-à-dire en dissolvant les conservateurs à chaud (60°C) à la dose de 1/10 000 dans une huile interestérifiée hydrodispersible (Labrafil - M 1944 CS) et en mesurant l'Ip chaque jour pendant 90 jours à 60°C. On obtient un profil de courbe Ip = f(t) comportant un maximum d'auto-oxydation net.

L'anti-oxydant est d'autant plus efficace que le maximum d'auto-oxydation est retardé.

Les résultats obtenus avec les trois conservateurs testés permettent d'avancer la séquence suivante:

Pouvoir anti-oxydant thiosalicylate de zinc> pyrithione zinc> acide thiosalicylique

2. Détermination des coefficients de partage à 20°C

| Produit | Système | Valeurs de P |
|---|---|---|
| Pyrithione zinc | Octanol-eau | 0,64 |
| | $CHCl_3$-eau | 0,30 |
| Thiosalicylate zinc | Octanol-eau | 0,21 |
| | $CHCl_3$-eau | 0,05 |

3. Solubilité maximum dans l'eau à 20°C

Déterminée par spectroscopie dans l'eau à 20°C:
Pyrithione zinc = 2 mg % ml
Thiosalicylate de zinc = 1,80 mg % ml.

Il est intéressant de disposer d'un produit peu hydrosoluble en général mieux toléré localement.

4. Teneur en zinc

| Composés | % Zinc |
|---|---|
| Pyrithione zinc | 20,5 |
| Thiosalicylate zinc (exemple 1) | 30 |
| Thiocrésotinate zinc (exemple 7) | 28,1 |
| Exemple 8 | 26,3 |
| Exemple 9 | 25,7 |

Les composés selon l'invention contiennent jusqu'à 50% de zinc en plus par rapport au pyrithione zinc.

Tolérance locale

Elle a été déterminée comparativement au pyrithione zinc pour les composés des exemples 1, 7, 8 et 9.

Pour chaque composé la tolérance a été recherchée sur 5 cobayes albinos adultes. L'application de produit 3 fois par semaine pendant 2 semaines sur le flanc préalablement rasé ne provoque aucune réaction.

La tolérance cutanée est pour 4 produits de l'invention meilleure qu'avec le pyrithione zinc.

Applications

Compte tenu de leur parfaite tolérance locale, de leur fort pouvoir anti-oxydant et de l'apport de zinc, ces molécules sont utiles en cosmétologie et plus particulièrement dans le traitement des pellicules, de la séborrhée et du vieillissement cutané.

A titre d'exemple non limitatif on donne ci-après quelques formulations dans lesquelles les composés selon la présente invention peuvent être utilisés seuls ou associés à d'autres composants doués de propriétés cosmétologiques dans les excipients appropriés.

Formule 1: shampooing
- Thiosalicylate de zinc 0,5 à 3%
- Alcoyl éthoxy sulfosuccinate de sodium
- Parfum hypoallergique
- Excipients pH 7±0,5.

Formule 2: shampooing sec
- Méthoxy-4-thiosalicylate de zinc 2±1,5%
- Excipient et tensio actif anionique

Formule 3: Crème traitement vieillissement cutané
- Thiosalicylate de zinc 1%
- Vitamine A ester glycérique
- Glycérine
- Prodermium (hydratant)
- Parfum naturel essence végétale
- Excipient émulsifiant

Formule 4: Lotion capillaire
- Thiosalicylate de zinc
- Excipient

Formule 5: Composition anti-acné
- Chloro-5-thiosalicylate de Mg 2 à 5%
- huile de maïs
- huile amande douce.

**Revendications**

1. Composition à usage externe pour le traitement et la prévention des désordres cutanés, notamment le traitement et la prévention des pellicules du cuir chevelu, caractérisée en ce qu'elle comporte à titre de principes actifs au moins un dérivé métallique de formule I:

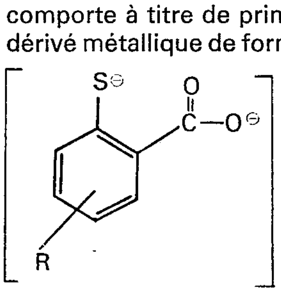

(I)

dans laquelle,
R est l'hydrogène, un halogène, un radical alcoyle inférieur en $C_1$ à $C_3$, un radical alcoxy inférieur en $C_1$ à $C_3$,
M représente les cations métalliques suivants: $Zn^{++}$, $Cu^{++}$, $Mg^{++}$, $Mn^{++}$, $Fe^{++}$, $Ni^{++}$ et $Ag^{+}$,
n représente les valeurs 1 ou 2.

2. Composition selon la revendication 1, caractérisée en ce que R est l'hydrogène, le chlore, un radical méthyle ou méthoxy.

3. Composition selon l'une des revendications 1 et 2, caractérisée en ce que le dérivé métallique de formule I est choisi parmi:
- le thiosalicylate de zinc,
- le thiosalicylate de manganèse,
- le thiosalicylate de nickel,
- le thiosalicylate de fer,
- le thiosalicylate d'argent,
- le thiosalicylate de cuivre,
- l'ortho thiocrésotinate de zinc,
- le méthoxy-4 thiosalicylate de zinc,
- le chloro-5 thiosalicylate de zinc.

4. Composition selon l'une des revendications 1 à 3, caractérisée en ce qu'elle est destinée au traitement du cuir chevelu.

5. Composition selon la revendication 4, caractérisée en ce qu'elle se présente sous forme de shampooing ou de lotion capillaire.

**Patentansprüche**

1. Zusammensetzung zur äusserlichen Verwendung für die Behandlung und Verhütung von Hautstörungen, insbesondere für die Behandlung und die Verhütung von Kopfhautschuppen, dadurch gekennzeichnet, dass sie als Wirkstoffe zumindest ein Metallderviat der Formel (I):

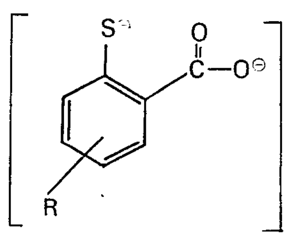

(I)

worin R Wasserstoff, Halogen, nied. Alkyl mit 1 bis 3 C-Atomen oder nied. Alkoxy mit 1 bis 3 C-Atomen bedeutet; M eines der folgenden Metallkationen darstellt: $Zn^{++}$, $Cu^{++}$, $Mg^{++}$, $Fe^{++}$, $Ni^{++}$ und $Ag^{+}$; und n der Wert 1 oder 2 ist, enthält.

2. Zusammensetzung nach Anspruch 1, dadurch gekennzeichnet, dass R Wasserstoff, Chlor oder eine Methyl- oder Methoxygruppe darstellt.

3. Zusammensetzung nach den Ansprüchen 1 und 2, dadurch gekennzeichnet, dass das Metallderivat der Formel (I) unter Zinkthiosalicylat, Manganthiosalicylat, Nickelthiosalicylat, Eisenthiosalicylat, Silberthiosalicylat, Kupferthiosalicylat, Zinkorthothiocresotinat, Zink-4-methoxythiosalicylat und Zink-5-chlorthiosalicylat ausgewählt ist.

4. Zusammensetzung nach den Ansprüchen 1 bis 3, dadurch gekennzeichnet, dass sie zur Behandlung der Kopfhaut bestimmt ist.

5. Zusammensetzung nach Anspruch 4, dadurch gekennzeichnet, dass sie in Form eines Shampoos oder Haarwassers ist.

## Claims

1. A composition for external use for the treatment and prevention of skin disorders, particularly for the treatment and prevention of dandruff, characterised in that it contains as an active principle at least one metallic derivative corresponding to formula I:

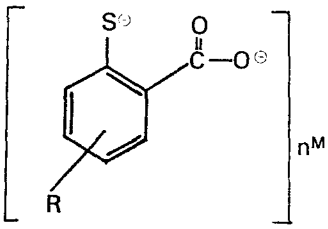

(I)

wherein
R represents hydrogen, a halogen, a $C_1$-$C_3$ lower alkyl radical, or a $C_1$-$C_3$ lower alkoxy radical,
M represents the following metallic cations: $Zn^{++}$, $Cu^{++}$, $Mg^{++}$, $Mn^{++}$, $Fe^{++}$, $Ni^{++}$ and $Ag^{+}$,
and n represents 1 or 2.

2. A composition according to claim 1, characterised in that R represents hydrogen, chlorine, a methyl radical or a methoxy radical.

3. A composition according to one of claims 1 and 2, characterised in that the metallic derivative corresponding to formula I is selected from among the following:
- zinc thiosalicylate,
- manganese thiosalicylate,
- nickel thiosalicylate,
- iron thiosalicylate,
- silver thiosalicylate,
- copper thiosalicylate,
- zinc orthothiocresotinate,
- zinc 4-methoxythiosalicylate, and
- zinc 5-chlorothiosalicylate.

4. A composition according to one of claims 1 to 3, characterised in that it is for use in treatment of the scalp.

5. A composition according to claim 4, characterised in that it is in the form of a shampoo or a capillary lotion.